# FASCICULE DE BREVET EUROPEEN

(11) **EP 2 393 978 B1**
(45) Date de publication et mention de la délivrance du brevet: **21.11.2012**
(21) Numéro de dépôt: 10707600.2
(22) Date de dépôt: 27.01.2010
(51) Int. Cl.: D06M 23/08, D06M 15/19, D06M 23/12, A61K 9/52, A61K 9/70, A61P 3/00, A61P 17/00, A61Q 13/00, A61Q 15/00, A61Q 19/06, A41D 31/00

(54) **PROCÉDÉ D'ENDUCTION DE MICROSPHÈRES SUR UN MATÉRIAU SOUPLE**
VERFAHREN ZUR AUFBRINGUNG VON MIKROKÜGELCHEN AUF EIN FLEXIBLES MATERIAL
METHOD FOR COATING MICROSPHERES ONTO A FLEXIBLE MATERIAL

(30) Priorité: 09.02.2009 FR 0950787
(43) Date de publication de la demande: 14.12.2011
(73) Titulaire: AB7 INNOVATION, 31450 Deyme (FR)
(72) Inventeur: CHELLE, René, F-31190 Grepiac (FR); MAKOUMBOU, Urbain, F-31270 Frouzins (FR); LAUTIER, Jean-Pierre, F-31670 Labege (FR)
(74) Mandataire: Morelle, Guy Georges Alain
(86) Numéro de dépôt international: PCT/FR2010/050127
(87) Numéro de publication internationale: WO 2010/089492

(56) Documents cités:
- EP-A- 1 600 210
- FR-A- 2 901 172
- FR-A- 2 908 427
- SIVAKUMAR M ET AL: "IN VITRO RELEASE OF IBUPROFEN AND GENTAMICIN FROM PMMA FUNCTIONAL MICROSPHERES" JOURNAL OF BIOMATERIALS SCIENCE. POLYMER EDITION, VSP, UTRECHT, vol. 13, no. 2, 1 janvier 2002 (2002-01-01), pages 111-126, XP008020096 ISSN: 0920-5063
- GOETZENDORF-GRABOWSKA B., KROLIKOWSKA H., GADZINOWSKI M.: "Polymer Microspheres as Carriers of Antibacterial Properties of Textiles: a Preliminary Study" FIBRES & TEXTILES IN EASTERN EUROPE, vol. 12, no. 4, 2004, pages 62-64, XP002537491

## Description

La présente invention a trait à l'application de matière sur un matériau souple. Elle concerne plus précisément l'enduction sans fusion, c'est à dire la mise en contact intime par fixation, de supports fibreux ou poreux tels que des textiles et non-tissés, par des microsphères polymériques chargées en une ou plusieurs substances actives.

Généralement, l'application de matière sur un matériau souple se fait par enduction ou par contrecollage. L'enduction consiste à enduire une surface plane a priori. Il existe plusieurs intérêts à procéder à l'enduction d'un support tel qu'un textile, celle-ci peut conférer différentes propriétés au tissu telle qu'une protection contre les liquides, les taches ou le feu, elle peut permettre de renforcer le support ou modifier son toucher. Le tissu, une fois enduit, peut être grainé pour lui donner différents aspects (mat, brillant, imitation cuir, etc...). La plupart des cas d'enduction connus sont réalisés dans l'objectif de renforcer, modifier ou adapter les caractéristiques physiques et mécaniques d'un textile ou d'un matériau souple pour une action ou une utilisation précise (Noël PONTHUS : « Textiles Techniques », WO/20008/080867 : « Method for preparing a woven, knitted or non woven fibrous substrate coated on one or both sides with at least one layer of reinforced elastomeric silicon », WO/2007/112982 : « Procédé d'enduction d'une surface textile », WO/2007/039763 : « Mélange caoutchouté de silicone liquide pour enduction textile », ESMERY CARON Structures : Architecture textile, Tentes et Bâches, CAPLP2/CONCOURS EXTERNE: ENTRETIEN DES ARTICLES TEXTILES : SESSION 2003).

Quel que soit le polymère utilisé : silicone, PVC, acrylique, polyuréthane, etc..., l'enduit se présente généralement sous la forme d'un liquide ou d'une pâte qui est réparti puis fixé par chauffage sur la surface du support (FR 2851265, A. Ponche et D. Dupuis : Rhéologie, Vol.2, 39-45 (2002) : « Relation entre rhéométrie et structure dans le cas des suspensions de dioxyde de titane dans les solutions de polymères »). Lorsque le polymère est solide, une opération de gélification (fusion/liquéfaction) a lieu avec polymérisation in situ (WO/0011061 (A1) : « Procédé d'enduction de textile », ARKEMA : LACOVYL® : Enduction - Mise en oeuvre du procédé). Les opérations de solubilisation ou de gélification des différents composites appellent l'utilisation de produits dits auxiliaires d'enduction et des solvants volatils qu'il faut éliminer par la suite et qui sont de ce fait source de problèmes environnementaux (179, UBA (Office fédéral de l'Environnement), 2001 : Enduction et contrecollage).

Le polymère le plus couramment utilisé pour l'enduction des textiles est le Polychlorure de vinyle (PVC) (ITF - Recherche et Développement : « Matériaux et Technologies » (1999), 63, GuT/ECA, 2000 : « Enduction d'envers des tapis ». Différents types d'enduction PVC sont ainsi décrits comme l'enduction PVC transparent (nappes intérieur ou extérieur, sets de tables), l'enduction PVC blanche (alèses), l'enduction PVC transparent ou de couleur avec grain (maroquinerie, sacs, trousses), l'enduction « Backcoating » (tissus d'ameublement), l'enduction PVC pour Textiles Techniques. Dans ce dernier cas, l'enduit type est constitué de 80% de PVC, 13% de Polyuréthane et 7% d'autres polymères et produits auxiliaires.

Les éléments polluants liés à la présence de produits auxiliaires sont généralement des monomères résiduels cancérigènes tels que l'acrylonitrile, le chlorure de vinyle, l'acrylamide, le 1,3-butadiène et le cyclohexène de vinyle, ainsi que les produits de décomposition provenant majoritairement des additifs.

Les dispositifs classiques utilisés dans les procédés d'enduction comprennent un cylindre (de transfert) ou une racle (couteau) qui tangente la surface du matériau à enduire afin d'appliquer une quantité définie (grammes ou cm3 par m2) d'enduit, ceci dans les cas d'enduction directe. L'enduction peut également être réalisée de manière indirecte, on la qualifie alors d'enduction transfert. Dans ce cas, l'enduit est appliqué préliminairement sur un autre support, la face enduite est ensuite contrecollée sur le matériau définitif (fragile en général).

D'autres types d'enduction sont également connus dans lesquels différents polymères et même des métaux sont appliqués sur des fibres ou des textiles. On entend par « textile enduit » un support textile additionné d'une résine polymère en quantité variant d'une fraction du poids du textile à plusieurs fois son poids. Toutes les matières textiles peuvent potentiellement être utilisées, cependant, la compatibilité entre la fibre et l'enduction doit être prise en compte. Les formulations d'enduction dans ces cas sont à base de polychlorure de vinyle, polyuréthanne, acrylique et élastomères naturels ou synthétiques. L'addition de plastifiants, charges minérales ou autres auxiliaires est nécessaire à la fixation des polymères et permet en outre de conférer des propriétés additionnelles spécifiques au matériau en vue d'un procédé ou d'un usage final donné.

On peut, par exemple, pratiquer le complexage qui se réalise par contre collage (ou laminage) sur un support textile de films, de mousses ou de membranes (microporeuses, en polyuréthanne (PUR) ou en Polytétrafluoroéthylène (PTFE)) conférant des fonctions barrières de respirabilité ou d'étanchéité au complexe.

On parle également de pré-imprégné dans le cas de structures textiles traitées avec une résine thermodurcissable non réticulée ou une résine thermoplastique. Ces structures textiles sont pré-imprégnées par des procédés en solution, en voie fondue, par poudrage, hybridation ou transfert. Lors d'un traitement thermique, le matériau pré-imprégné est conformé dans un moule.

De manière générale, les produits d'enduction et de contrecollage textiles peuvent être distingués selon leur composition chimique en cinq familles principales.

Les produits d'enduction sous forme de poudres, à base de□
- polyoléfines (en particulier de polyéthylène),
- polyamide 6,□
- polyamide 6.6,
- copolyamides,
- polyester,
- polyuréthanne,
- chlorure de polyvinyle,
- polytétrafluoroéthylène.

Les produits d'enduction sous forme de pâtes, à base des polymères mentionnés ci-dessus, et contenant également des adjuvants tels que :
- dispersants (tensioactifs, souvent des éthoxylates d'alkylphénol),
- agents solubilisants (glycols, N-méthylpyrrolidone, hydrocarbures),
- agents moussants (huiles minérales, acides gras, sels d'ammoniaque d'acide gras),
- adoucissants (en particulier phtalates, sulfonamides),
- épaississants (polyacrylates),
- ammoniaque.

Les produits d'enduction sous forme de dispersions de polymères (formulations aqueuses), contenant approximativement 50 % d'eau, à base de :
- polyméthacrylate de (butyle, éthyle, méthyle, etc.),
- acide polyacrylique,
- polyacrylonitrile,
- polyacrylamide,
- 1,3-polybutadiène,
- polystyrène,
- polyuréthanne,
- chlorure de polyvinyle,
- polyvinylacétate,
- et les copolymères des polymères ci-dessus mentionnés.

Des adjuvants sont également présents, comme dans les pâtes d'enduction.

Les produits d'enduction sous forme de résines mélamine, produits par la réaction de la mélamine et du formaldéhyde et par l'éthérification ultérieure principalement avec le méthanol en milieu aqueux (teneur en eau de 50 à 70 %).

Les produits d'enduction sous forme de dispersions de polymères (formulations à base d'un solvant organique), à base de polyuréthanne et de silicones dispersés dans un solvant organique.

Dans tous les cas, quelle que soit la nature du produit d'enduction, une étape de chauffage est nécessaire pour permettre l'application du polymère sur le support. Cette étape vise à porter le polymère à son état de fusion avancée de manière à ce que la structure interne du polymère soit altérée et permette une liaison intime avec les fibres du support. Elle est réalisée par des fours ou fours-tunnels bien connus de l'homme du métier.

Selon le type de produits d'enduction, les problèmes environnementaux liés aux émissions dans l'air de produits toxiques ne sont pas les mêmes. Ils peuvent survenir directement lors de l'utilisation du polymère lui-même comme dans le cas des produits d'enduction sous forme de poudres avec l'utilisation du polyamide 6 et ses copolymères (le monomère résiduel e-caprolactam se dégage aux températures normales du procédé).

Ils peuvent également survenir de manière indirecte, mais incontournable, par l'utilisation d'additifs dont la présence est nécessaire, comme dans le cas des produits d'enduction sous forme de pâtes. Les produits toxiques dégagés dans ce cas sont principalement :
- des alcools gras, acides gras, amines gras, provenant des tensioactifs,
- des glycols provenant d'émulsifiants,
- des alkyl phénols provenant des dispersants,
- des glycols, hydrocarbures aliphatiques, N-méthylpyrrolidone des agents hydrotropiques,
- des hydrocarbures aliphatiques, acides gras/sels, ammoniaque provenant des agents moussants,
- des phtalates, sulfonamides/esters des adoucissants/plastifiants,
- des acides acryliques, acrylates, ammoniaque, hydrocarbures aliphatiques provenant des épaississants.

En ce qui concerne les produits d'enduction sous forme de dispersions de polymères (formulations aqueuses), les composants qui sont responsables des émissions dans l'air sont les dispersants, les composants résiduaires de la polymérisation (en particulier, le t-butanol utilisé comme catalyseur dans les réactions de polymérisation radicalement initialisées) et les monomères découlant de la réaction incomplète pendant la polymérisation. Ces derniers sont particulièrement importants en ce qui concerne les nuisances dans l'air ambiant du lieu de travail et des odeurs. Ils incluent :
- les acrylates tels que l'acide acrylique, l'acrylate de butyle, l'acrylate d'éthyle, l'acrylate de méthyle, l'acrylate d'éthylhexyle et l'acétate de vinyle,
- les monomères cancérigènes tels que l'acrylonitrile, le chlorure de vinyle, l'acrylamide, le 1,3-butadiène et le cyclohexène de vinyle.

Le cyclohexène de vinyle n'est pas souvent identifié dans les rejets gazeux. Toutefois, il se forme toujours (2 + 2 produits de cyclo-addition) si l'on utilise du 1,3-butadiène. L'acrylamide dans les rejets gazeux est souvent lié aux émissions de formaldéhyde (produits de réaction du méthylol-acrylamide).

Les produits d'enduction sous forme de résines mélamine peuvent contenir des quantités considérables de formaldéhyde libre et de méthanol. Pendant leur application, la réaction de réticulation de la résine avec elle-même ou avec le tissu (par exemple, le coton) est initiée par un catalyseur acide et/ou la température en libérant des quantités stoechiométriques de méthanol et de formaldéhyde.

Les produits d'enduction sous forme de dispersions de polymères (formulations à base de solvants organiques), bien que peu courants dans l'industrie de finition des textiles, nécessitent l'utilisation d'un matériel de traitement des rejets gazeux incluant une oxydation thermique ou l'adsorption sur charbon actif. En effet, cette technique oblige un passage dans un four pour polymériser le composite et éliminer les solvants volatils avant refroidissement et enroulement.

Il apparaît donc, quelle que soit la famille de produits d'enduction utilisée, qu'un certain nombre de composés toxiques sont émis directement ou indirectement lors des procédés d'enduction classiquement mis en oeuvre.

La présente invention a pour but de pallier les inconvénients de l'art antérieur et propose un procédé d'enduction de matériaux souples, non tissés ou textiles par thermo-application de microsphères polymériques chargées en une ou plusieurs substances actives, sans qu'aucune émission toxique pour l'homme ou l'environnement ne survienne durant la mise en oeuvre. Ce résultat est obtenu par la mise au point d'un procédé permettant la fixation de certains polymères sélectionnés pour leurs propriétés physico-chimiques et structurales, non polluants et chargés en substance active, se présentant sous la forme de microsphères, aux fibres ou aux pores du support, sans recourir à l'utilisation de produits auxiliaires entraînant l'émission dans l'air de produits toxiques.

Cette technique permet de conférer des propriétés actives au matériau souple qui est enduit à l'issue du procédé objet de la présente invention, ceci grâce aux différentes propriétés des substances actives incorporées dans les microsphères polymériques. Dans la suite de cette description, l'expression « substance active » sera utilisée dans le cadre de l'invention au singulier par commodité syntaxique mais aura aussi bien la portée d'une seule substance ou composition active que plusieurs substances ou compositions actives mélangées entre elles au sein d'une même microsphère ou bien encore mélangées par l'association de plusieurs microsphères chargées chacune en une substance active.

L'activité apportée au matériau souple par l'intermédiaire des microsphères chargées résulte de la libération de la substance active emmagasinée au sein des microsphères par diffusion à travers le réseau microporeux de celle-ci. Le principe de l'invention réside en effet dans la préservation de la structure poreuse des microsphères, ceci malgré le traitement thermique qui est appliqué aux polymères constituants des microsphères au cours du procédé d'enduction. De manière surprenante, par le choix de certains polymères spécifiques et dans une gamme de températures précises, il a été constaté qu'il était possible d'obtenir une fixation intime et solide des microsphères chargées aux fibres ou aux pores du matériau souple, par thermo-application, sans utiliser de produits auxiliaires d'enduction ou de plastification, tout en préservant la structure du réseau poreux des microsphères.

Le procédé objet de la présente invention permet d'obtenir un matériau souple activé qui constitue pour l'utilisateur une alternative :
- à la prise classique de certaines substances actives par voie orale qui présente un bon nombre d'inconvénients comme le premier passage hépatique et sa série d'effets
- ou à la voie topique qui présente l'inconvénient d'avoir à appliquer plusieurs fois dans la journée une pommade ou un gel contenant la substance active, avec souvent l'inconvénient de taille de ne pouvoir disposer d'un lieu pour le faire décemment et correctement.

En mettant en contact avec sa peau le matériau souple traité selon le procédé objet de l'invention, l'utilisateur bénéficie d'une libération continue et prolongée de la substance active emmagasinée dans les microsphères qui imprègnent les fibres du tissu. Dans le cas de substances actives telles que des principes actifs à effet thérapeutique, ceci présente l'avantage de faciliter les prises de traitement, non seulement en terme d'administration et de libération vers la cible du principe actif, mais également en terme de diffusion et de régularité des prises incombant à l'utilisateur.

De manière plus précise, la présente invention concerne un procédé d'enduction sans fusion de microsphères polymériques chargées en une ou plusieurs substances actives sur un matériau souple caractérisé en ce qu'il comprend les étapes suivantes□
a) sélectionner un polymère réticulé poreux,
b) incorporer une composition lipophile liquide à la température ambiante, comprenant au moins une substance active, dans le polymère par chauffage à une température supérieure de 1 à 5 °C à la température de transition vitreuse du polymère afin d'obtenir des microsphères chargées,
c) disposer les microsphères chargées sur un matériau souple,
d) chauffer le matériau souple comprenant les microsphères chargées à une température supérieure de 1 à 10 °C à la température de fusion du polymère, sans atteindre l'état de fusion du polymère,
e) obtenir le refroidissement du matériau souple imprégné de microsphères chargées.

La nature des polymères utilisés pour former les microsphères est décisive pour le bon déroulement du procédé selon l'invention. Ceux-ci doivent en effet présenter une structure réticulaire de manière à retenir les substances actives qui leur sont associées et ainsi pouvoir les emmagasiner afin de former les microsphères. Ils doivent également présenter un réseau poreux se caractérisant par une pluralité de micropores interconnectés pour permettre la diffusion des substances actives après le procédé en lui-même d'enduction du support cible, ceci pour permettre la libération dans le milieu extérieur et/ou la peau de l'utilisateur qui porte ledit support.

Dans le cadre de l'invention, les microsphères sont constituées d'un seul type de polymère réticulé poreux. De préférence, les polymères réticulés poreux choisis pour former les microsphères sont des polyamides, des polyéthers block amides ou des éthylènes vinyle acétate. Plusieurs microsphères constituées de différents polymères choisis parmi les polymères réticulés poreux précités peuvent être mélangées. A titre indicatif, de telles microsphères ont un diamètre compris entre 5 et 200 µm, avec une structure interne pleine hétérogène, constituée d'une pluralité de micropores interconnectés les uns aux autres et d'un diamètre d'environ 0,1 à 10 µm.

Il est connu des procédés d'enduction de fibres textiles faisant intervenir des microcapsules. Les microcapsules peuvent être constituées de toutes sortes de polymères et se caractérisent par une structure creuse, venant envelopper comme une membrane la composition active incorporée dans le coeur de la microcapsule. Les procédés de fabrication et de chargement de ces microcapsules sont complexes et coûteux. Il est difficile d'emmagasiner des quantités importantes de composition active à l'intérieur des microcapsules. De plus, la composition emmagasinée ne diffuse pas de manière progressive, elle est libérée en masse lors de la dégradation de la membrane constituant l'enveloppe externe de la microcapsule par rupture mécanique ou dégradation chimique par un solvant tel que l'eau. En général, ces microcapsules ne sont pas fixées directement sur le support et nécessitent l'utilisation de liants ou d'autres produits auxiliaires. Une étape de chauffage du polymère d'enduction est réalisée à des températures élevées pour améliorer la fixation des microcapsules par fusion sur les fibres du support ce qui peut provoquer une dégradation de la composition active emmagasinée ainsi qu'une libération prématurée de celle-ci par rupture de la paroi de la microcapsule. Parfois, le liant utilisé se mélange à la matrice polymérique de la microcapsule (fixation sur le support), ce qui provoque une imperméabilisation de la microcapsule et freine la diffusion de la composition active restante. Ce type de textile enduit avec des microcapsules se révèle au final peu efficace en terme de diffusion de la composition active emmagasinée et en terme de résistance au lavage étant donné la fragilité inhérente à la structure des microcapsules et leur mauvaise fixation aux fibres du support.

Les substances actives pouvant être utilisées dans le cadre du procédé objet de l'invention sont constituées par toute sorte de composés présentant un effet de nature thérapeutique, dermo-cosmétique ou olfactive. Afin de faciliter leur incorporation dans le ou les polymères réticulés poreux constitutifs des microsphères, les substances actives sont associées à des compositions lipophiles liquides à température ambiante. Le caractère lipophile de la composition facilite non seulement l'incorporation de la substance active au sein de la microsphère, mais il permet également d'en améliorer la libération à partir des micropores, une fois la microsphère fixée sur les fibres ou pores du support, et améliore la diffusion progressive au contact de la peau grâce aux interactions lipophiles résultant du sébum.

De préférence, la substance active est choisie parmi des molécules à action :
- thérapeutique (i.e., anti-douleur, anti-inflammatoire, veinotonique, ...);
- dermo-cosmétique (i.e., amincissante -lipolytique et/ou drainante-, hydratante, apaisante, anti-âge -antioxydante et/ou restructurante et/ou réparatrice-, éclaircissante, jambes lourdes);
- olfactive (i.e., anti-stress, désodorisante/odorisante, attractive et/ou répulsive).

Elle est associée à une composition lipophile liquide à température ambiante qui peut être formulée à partir d'huiles essentielles, d'essences naturelles ou de synthèse, de substances liquides ou solubilisées en milieu lipophile, naturelles ou de synthèse.

L'incorporation en elle-même de la substance active associée à la composition lipophile liquide à température ambiante au sein de la microsphère est réalisée par simple chauffage du polymère réticulé poreux à une température supérieure de 1 à 5° Celsius, de préférence 3° Celsius, à la température de transition vitreuse dudit polymère de manière à permettre une incorporation dans le polymère sans dénaturer la structure du réseau microporeux de celui-ci. L'état de fusion du polymère n'est pas atteint, les propriétés des substances actives sont préservées. Aucun additif technique de plastification n'est utilisé. Cette étape est effectuée en un temps suffisamment court pour ne pas altérer les propriétés physico-chimiques du polymère ; elle durera moins de 5 minutes, de préférence 3 minutes.

La ou les substances actives sont incorporées au polymère réticulé poreux dans un rapport pondéral représentant 1 à 40 % en poids de la microsphère chargée résultante. Plusieurs substances actives de nature différente peuvent ainsi être incorporées dans un type de microsphères. Les quantités de substance active emmagasinées dans les microsphères sont plus importantes que lors du chargement des microcapsules décrit dans l'art antérieur.

L'étape suivante consiste à disposer les microsphères chargées en une ou plusieurs substances actives sur le matériau souple. Pour cela, les microsphères sont réparties manuellement ou de manière automatisée sur la surface du matériau souple. Celui-ci peut être préalablement disposé dans tout type de dispositif industriel permettant la mise en oeuvre du procédé d'enduction de manière industrielle en vue d'obtenir des rendements de production importants tels que des convoyeurs ou autre forme de chaîne automatisée. Il est possible de moduler l'intensité de diffusion des substances actives emmagasinées en répartissant des concentrations importantes de microsphères sur le matériau souple.

Le matériau souple est constitué par un support fibreux ou poreux. Il comprend une pluralité de fibres tel qu'un tissu ou autre textile, mais peut également être constitué de n'importe quel type de structure non tissée sur laquelle les microsphères chargées sont susceptibles d'être disposées et fixées par thermo-application. Il est également envisageable de réaliser le procédé objet de la présente invention sur un support ne présentant pas une structure fibreuse mais dont l'aspect de surface ou la structure extérieure permet aux microsphères de se répartir et de se fixer telle qu'une structure poreuse. De préférence, le matériau souple sera choisi parmi des textiles tissés ou non tissés composés de fibres naturelles d'origine animale ou végétale, ou encore des fibres synthétiques.

L'ensemble du matériau souple comprenant les microsphères chargées est ensuite chauffé à une température supérieure de 1 à 10 ° Celsius à la température de fusion du polymère réticulé poreux constituant les microsphères. De manière surprenante, il a été observé qu'il était possible de fixer les microsphères aux fibres ou aux pores du support par thermo-application, ceci dans une gamme de températures précises, qui dépend du polymère constitutif de la microsphère, sans endommager la structure réticulée poreuse de la microsphère, l'état de fusion du polymère n'étant pas atteint. Cette étape est en effet réalisée en un temps suffisamment court pour préserver les propriétés physico-chimiques des polymères réticulés poreux utilisés pour l'élaboration des microsphères et permettre la diffusion des substances actives à travers le réseau microporeux interconnecté des microsphères. De préférence, elle est comprise entre 1 à 10 secondes. La présence de la substance active à l'intérieur des microsphères semblerait jouer un rôle dans la fixation des microsphères sur les fibres ou pores du support à des températures précises et dans un temps donné sans que l'état de fusion du polymère ne soit atteint, la substance active rabaissant la viscosité générale de la microsphère chargée.

Si plusieurs types de polymères sont utilisés dans l'élaboration des microsphères, plusieurs étapes de chauffage peuvent être prévues dans le procédé d'enduction. Chaque étape est ajustée selon une température supérieure de 1 à 10 ° Celsius à la température de fusion du polymère réticulé poreux considéré. On commence dans ce cas par les polymères présentant la plus haute température de fusion pour finir avec ceux dont la température de fusion est la plus faible pour ne pas détériorer leur structure par fusion. Ceci permet notamment de combiner les propriétés de relargage plus ou moins lente ou rapide des polymères utilisés pour la fabrication des différentes microsphères.

Le chauffage peut être obtenu par tout type de dispositif permettant une production industrielle de matériaux souples enduits, spécialement des fours, blocs de chauffage avec rouleaux applicateurs ou presses. Si une force de pression est exercée conjointement au chauffage, le temps de chauffage ainsi que la température utilisée sont réduits. Dans ce cas, il est même possible d'obtenir une fixation des microsphères en un temps très court, qualifiable de « temps flash », et à des températures inférieures à la température de fusion du polymère, ce qui présente un grand intérêt en terme de préservation des propriétés tant du polymère que de la substance active emmagasinée.

La dernière étape consiste à obtenir le refroidissement du matériau souple recouvert de microsphères chargées. De préférence, il sera obtenu au moyen d'un dispositif de refroidissement prévu à cet effet.

La présente invention permet d'obtenir des matériaux souples tels que des supports fibreux ou poreux chargés en une ou plusieurs substances actives qui vont diffuser dans l'environnement immédiat de l'utilisateur de manière continue avec des effets pics prolongés par une diffusion d'entretien selon les polymères sélectionnés pour réaliser les microsphères. Aucun produit auxiliaire n'est utilisé pour le plus grand confort et la santé de l'utilisateur. Les matériaux souples fabriqués selon le procédé objet de l'invention peuvent comporter une ou plusieurs couches de microsphères chargées pour cumuler les effets de plusieurs substances actives. Ils peuvent concerner des vêtements entiers que l'utilisateur va porter au contact direct de sa peau comme des sous-vêtements, des collants, des gaines, ceintures ou des maillots de corps par exemple, mais également tout type d'accessoire textile ou poreux que l'utilisateur est susceptible de porter. Ils peuvent également concerner des parties seulement de vêtement pour obtenir une diffusion plus localisée à certaines zones du corps. Les vêtements en question peuvent être utilisés pour la diffusion de substances actives, en contact direct ou non avec la peau de l'utilisateur.

Les exemples qui suivent sont donnés à titre d'illustration seulement de la présente invention et ne doivent pas être interprétés de manière restrictive quant à sa portée.

### EXEMPLES

### ELABORATION DES MICROSPHERES

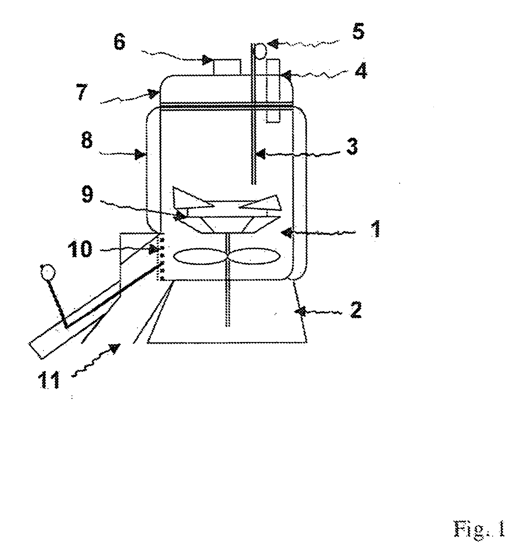

L'élaboration des microsphères se fait dans un dispositif tel que représenté selon la figure 1, se composant d'une cuve de mélange 1, reposant sur un socle 2, d'une sonde de température 3, d'une alimentation pour les liquides 4, d'un thermomètre 5 relié à la sonde 3, d'une partie visionneuse 6 présente sur le couvercle 7 de la cuve, d'une enveloppe de chauffage 8, d'un système d'agitation 9 et d'un sas de vidange 10 permettant de récupérer les microsphères qui se présentent dans le déversoir 11.

Les substances actives sont chargées dans les différents polymères selon la technologie bien connue par le demandeur (FR 2 901 172, AB7 Industries)
- On charge un granulé micronisé (de 5 à 200 □m de diamètre) de polyamide (ORGASOL), de polyéther block amides (PEBAX), de copolymère d'éthylène et d'acétate de vinyle (EVA) ou encore d'autres polymères réticulés poreux en Principes Actifs par incorporation à basses températures entre 20 et 95°C, de préférence à 70°C.
- Ce granulé dit polymère porteur (microsphère), c'est-à-dire celui dans lequel les Principes Actifs sont incorporés, doit être de la famille des polymères réticulés « poreux », capables d'emmagasiner par séquestration des Principes Actifs liquides lipophiles. Le polymère doit être solide à la température ambiante et doit avoir une température de transition vitreuse (Tg) comprise entre 30 et 95°C. De même son point de fusion doit être compris entre 75 et 180°C, mais de préférence inférieur à 140°C. Ce polymère devra être en mesure de relarguer presque la totalité des Principes Actifs qu'on y aura incorporés.
- Les Principes Actifs ou les solutions de Principes Actifs et excipients doivent être de nature lipophile et peuvent représenter jusqu'à 40 % en poids du produit fini. Ce sont des huiles essentielles naturelles, des essences naturelles ou de synthèse, des substances liquides ou solubilisées en milieu lipophile, qui soient naturelles ou de synthèse, des médicaments, des parfums, etc.
- L'incorporation des Principes Actifs dans le polymère se fait par voie thermique, soit 1°C à 5°C au dessus de la température de transition vitreuse du polymère, de préférence 3°C. Elle se fait en un temps court de moins de 5 minutes, de préférence en 3 minutes, sans additifs techniques de plastification. Le refroidissement termine l'incorporation et assure la stabilité des Principes Actifs incorporés.
- L'opération de réalisation des microsphères chargées se déroule comme suit :
   - dans la cuve 1 d'un mélangeur rapide (compoundeur) de laboratoire par exemple du type de celui de la marque GUNTHER PAPENMEIER KG, d'un volume utile de 8 litres on introduit la quantité de polymère à charger, préchauffée à la température de travail par de l'eau chaude ou tout autre fluide circulant dans l'enveloppe 8 ;
   - on ferme le couvercle 7 pour commencer la mise en température d'incorporation du polymère, sous agitation à l'aide du système d'agitation 9 ;
   - à température fixée, on introduit toujours sous agitation les Principes Actifs (PAs) liquides par l'alimentation 4 pendant 2 à 5 minutes ;
   - on garde la température de réaction pendant 3 minutes avant de refroidir par l'enveloppe 8 toujours sous agitation ;
   - puis on ouvre le sas de vidange 10 pour recueillir les microsphères chargées par le déversoir 11.

### ENDUCTION DES MICROSPHERES SUR LE MATERIAU SOUPLE

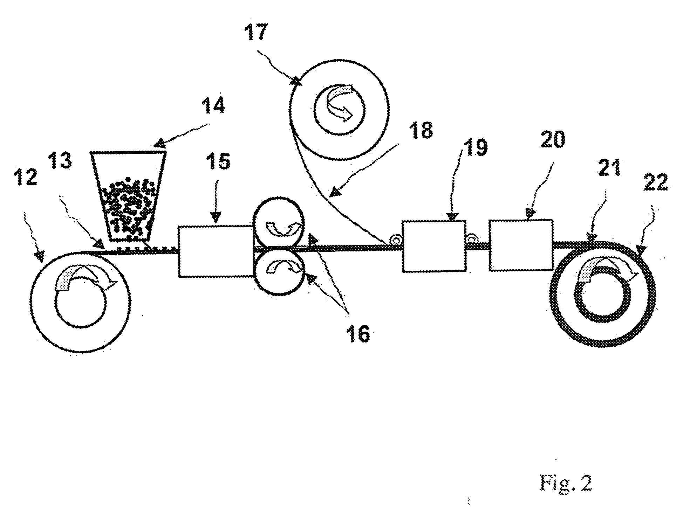

L'enduction se fait dans un dispositif tel que représenté selon la figure 2, se composant d'un rouleau dérouleur 12, le matériau souple à traiter est matérialisé par la référence 13, d'une trémie à microsphères 14, d'un bloc chauffage 15, de rouleaux de pressage 16, d'un autre rouleau dérouleur 17, le matériau souple à appliquer sur l'enduit est matérialisé par la référence 18, d'un bloc chauffage avec rouleaux applicateurs 19, d'un bloc de refroidissement 20, le matériau enduit est représenté par la référence 21, d'un rouleau de bobinage du matériau enduit 22.

L'enduction se déroule comme suit :
A. dépose des microsphères (~5 à 200 □m de diamètre) à partir de la trémie 14, librement ou dans un moule, sur le matériau souple 13 déroulé de sa bobine 12 ;
B. chauffage dans la cellule de chauffage 15 à la température correspondant à la matrice des microsphères ;
C. pressage de l'enduit sur le matériau à l'aide des rouleaux de pressage 16 ;
D. application (éventuellement) du 2^{ème} matériau souple 18 déroulé de sa bobine 17 sur l'enduit dans le dispositif applicateur chauffant 19 ;
E. refroidissement de l'ensemble dans la cellule de refroidissement 20 ;
F. bobinage du matériau enduit 21 sur la bobine 22.

Le matériau enduit peut être mis en forme de diverses manières :
a. par simple découpage ou par découpage et couture ;
b. par application d'un film barrière sur la surface enduite pour obliger le relargage du principe actif par l'autre face, avant découpage ou découpage et couture. En effet, on peut faire en sorte que ce ne soit pas la face enduite qui soit au contact de la peau, mais l'autre face, ce qui évite de mettre le polymère au contact direct de la peau pour les zones sensibles du corps comme le visage ;
c. on peut également recouvrir la face enduite par du textile.

### Exemple 1

Des essais d'enduction de polymère actif sous forme microsphérique sur du textile ont été réalisés pour vérifier le relargage du principe actif.

L'actif est un parfum relaxant, ELISIA, fourni par ROBERTET, incorporé dans des microsphères en polyamide, l'ORGASOL 2002 EXD NAT 1 d'ARKEMA dont le point de fusion est de 177°C, selon la méthode du demandeur décrite plus haut.

Le textile utilisé est en coton tissé, extensible à 180% dans un sens et à 36% dans l'autre, fourni par un industriel du textile, ROULEAU GUICHARD. Pour faciliter la perception des apports et des pertes, nous avons appliqué de grandes quantités de microsphères actives :
□ Les microsphères sont chargées à 25% de parfum ELISIA, un parfum relaxant,
□ On découpe des morceaux de textile de 20 cm X 20 cm,
□ On étale 1 g de microsphères chargées sur chaque morceau de textile, soit 2,5 mg/cm² comportant 0,625 mg de parfum,
□ On pose du papier sulfurisé sur les microsphères étalées sur le textile,
□ On applique, à défaut de dispositif de chauffage avec rouleaux de pressage, un fer à repasser à 180°C pendant 4 secondes sur le papier sulfurisé. On obtient le thermocollage des microsphères sur le textile sans qu'il y ait fusion réelle des microsphères, sans apport d'aucune substance supplémentaire d'enduction. Les microsphères sont collées entre elles et aux fibres. En fonction de la quantité étalée sur le textile, elles peuvent former une couche invisible ou une couche visible souple.
□ Dans le cas présent, le textile enduit de microsphères au parfum ELISIA en couche souple est soumis à observation sur le pouvoir d'évaporation du parfum à température ambiante et à 40°C en étuve. Nous obtenons :
   - A température ambiante on constate une libération moyenne de parfum de 2% par jour.
   - A 40°C en étuve la libération moyenne de parfum est de 4% par jour.

Nous en concluons que non seulement le relargage du principe actif n'est pas gêné, mais aussi que le comportement du polymère vis-à-vis de la température n'est pas modifié. En chauffant à 40°C, on dilate les pores qui permettent une libération plus élevée de parfum car le micro-réseau de ces pores n'est pas gravement perturbé par le mode d'application des microsphères.

Contrairement à l'enduction courante qui demande l'utilisation d'un polymère d'enduction (liant) dans lequel les microcapsules actives sont emprisonnées, ce qui gêne le relargage du principe actif, le procédé selon la présente invention donne un rendement bien supérieur. Cette gêne de relargage du principe actif est aussi constatée dans le cas des procédés par inclusion. Seule la liaison ionique semblerait se rapprocher de ces résultats, mais ce système présente la difficulté de charger négativement le textile et positivement les microcapsules, ce qui tranche avec la simplicité du procédé objet de la présente invention.

### Exemple 2

Dans cet exemple nous présentons le cas d'un principe actif qui exige une certaine formulation avant l'élaboration des microsphères. Il s'agit de la caféine que nous voulons appliquer par voie topique pour une action amincissante, lipolytique et drainante.

Cette substance présente le désavantage de n'être que très peu soluble. Elle recristallise en se déshydratant, ne pouvant plus dans ce cas passer la barrière dermique. Pour cela, la caféine est formulée avec un agent dispersant, un émulsionnant, un émollient et de l'eau. Le taux de caféine est de 3% dans la formulation totale. Ces substances de formulation ne présentent aucun inconvénient ni sanitaire, ni toxicologique, puisqu'elles sont autorisées et utilisées normalement dans l'élaboration des produits traitants.

Deux polymères ont été utilisés séparément pour l'élaboration des microsphères, afin d'en comparer les rendements. Ce sont d'une part l'ORGASOL 2002 EXD NAT 1 d'ARKEMA, qui est un polyamide qui donne des microsphères de 8 □m à 12 □m de diamètre dont le point de fusion est de 177°C, et d'autre part l'EVA ALCUDIA PA-541 de REPSOL YPF qui est un copolymère d'Ethylène et d'Acétate de vinyle qui donne des microsphères de 80 □m de diamètre et dont le point de fusion est de 85-90°C.

On prend une pièce de textile de 750 cm² sur laquelle on étale 15.000 mg de microsphères chargées, soit 20 mg/cm². L'enduction est réalisée comme décrit dans l'exemple 1, selon les conditions d'application de 180°C pendant 4 secondes pour l'ORGASOL et 100°C pendant 2 secondes pour l'EVA.

Pour accélérer le relargage du principe actif et seulement à titre comparatif, nous avons procédé au lavage des échantillons comme suit :
□ prélever des morceaux de chaque échantillon de tissu ;
□ préparer un liquide lavant à base d'eau et de détergent dans des éprouvettes fermées ;
□ plonger chaque morceau d'échantillon dans une éprouvette (3 morceaux différents par échantillon), fermer et placer dans une étuve à carrousel tournant pour l'agitation des éprouvettes;
□ lorsque l'étuve est pleine, la mettre en marche avec le chauffage à 30°C pendant 30 minutes ;
□ à l'arrêt de l'étuve, sortir les éprouvettes, les agiter vigoureusement (10 va-et-vient avec la main), les vider du liquide lavant, le remplacer par de l'eau propre, agiter et recommencer le rinçage 4 fois ;
□ sortir les échantillons et les mettre à sécher à l'air et les peser ;
□ procéder à l'extraction de la caféine restant sur l'échantillon pour en doser la quantité par chromatographie.

Les libérations comparées de caféine sont de 63% dans l'EVA et 95% dans l'ORGASOL. Ceci nous conduit à tirer les conclusions suivantes :
- Les microsphères à haute température de fusion sont celles qui relarguent le plus d'actif parce qu'ayant subi moins de transformation structurelle lors de l'enduction à cause de la faible différence entre leur point de fusion et la température d'application.
- Les polymères sont plus réactifs lorsque leur microstructure matricielle constituée d'un micro-réseau de pores n'est pas perturbée.
- Ce système d'enduction de microsphères sans fusion réelle de la matière présente un gros avantage sur les méthodes habituelles d'enduction qui procèdent par fusion totale du polymère ou par collage d'un film polymère obtenu par extrusion, donc par fusion totale. Le polymère fondu a un pouvoir de relargage nettement plus faible à cause de l'organisation de sa microstructure qui offre peu de continuité du réseau des micropores.

### Exemple 3

Les microsphères à la caféine en EVA, déjà évoquées dans l'exemple 2, sont appliquées sur un textile extensible, comme déjà décrit dans les exemples 1 et 2, pour une utilisation comme ceinture amincissante (via une action lipolytique et/ou drainante) .

La ceinture mesure 120 cm de long et 15 cm de large, soit une surface de 1.800 cm². Les microsphères sont thermocollées à raison de 19 mg/cm², soit 0,57 mg/cm² de caféine. On applique une température de 100°C pendant 2 secondes, de manière à ce que la température de fusion de 84°C ne soit pas réellement atteinte dans la masse du polymère.

Trois cas de tests ont été réalisés :
- La ceinture portée directement contre la peau pendant 20 jours,
- La ceinture portée sur des vêtements sans contact avec la peau pendant 20 jours,
- La ceinture non portée pendant 20 jours.

Il ressort de ces tests que :
- Le porteur de la ceinture a perdu 3 à 4 cm de tour de taille.
- La ceinture portée pendant 20 jours contre la peau a perdu en moyenne 55% de caféine, soit en moyenne 0,04 mg/cm² par jour. Cette donnée associée à la précédente met à l'évidence que la caféine a bien été relarguée pour réaliser son action.
- La ceinture portée par-dessus les vêtements sans contact avec la peau présente une perte de 5% de caféine. Celle-ci est sans doute perdue essentiellement par frottements.
- La ceinture non portée et laissée à l'air libre n'a pas perdu de caféine, mais au contraire a gagné de l'humidité.

Ce mode d'enduction permet de préserver à la fois les caractéristiques du textile et le bon fonctionnement des microsphères comme réservoirs de principes actifs pouvant être relargués de manière contrôlée.

### Exemple 4 :

A partir des échantillons de textile enduit avec des microsphères à la caféine en EVA comme décrit dans l'exemple 3, on découpe des morceaux de 20 cm² que l'on applique comme patches à l'aide d'un film collant.

Le constat sur 20 jours fait selon les mêmes conditions analytiques que dans l'exemple 3 est que 79% de caféine ont été relargués par le dispositif, soit 24% de plus pour la même surface que la ceinture.

Le fait que la face extérieure soit protégée par un film qui ne permet le transfert que dans un sens, favorise et accentue le transfert de la caféine de l'enduit vers la peau.

### Exemple 5 :

Dans cet exemple les microsphères utilisées sont chargées superficiellement par adsorption du mélange actif de niveau cosmétique, composé de caféine, d'un solvant cosmétique, d'un émulsifiant cosmétique, d'un gélifiant cosmétique et d'un émollient cosmétique, sur de la poudre de RILSAN® B (polyamide 11) de ARKEMA, dont le point de fusion est à 175°C, comme suit :
□ Préchauffer dans le compoundeur le RILSAN® (68%) et le CARBOPOLE ULTREZ 21 (1%)
□ Solubiliser la CAFEINE (10%) à 70°C dans de l'Eau (5%), du Glycérol (5%), de l'EUMULGIN SMS 20 (1%) et de l'EUTANOL G (10%)
□ Verser la solution chaude dans le compoundeur
□ Refroidir et sortir la poudre obtenue de microsphères actives.

Les microsphères actives sèches sont déposées entre deux couches de coton dont l'adhésion est assurée par hydrovaporisation et thermopressage entre 120°C et 150°C, de préférence à 130°C pendant 1 seconde.

Ce coton dit actif est ensuite conditionné sous forme de carrés ou de ronds.

Il est utilisable par application sur la peau après l'avoir aspergé d'une lotion démaquillante qui solubilise instantanément le mélange actif adsorbé sur le RILSAN® B et qui passe ainsi sur la peau.

Ce mode d'application du principe actif ne serait pas efficacement réalisable si l'on avait pratiqué une enduction normale.

## Revendications

1. Procédé d'enduction sans fusion de microsphères polymériques chargées en une ou plusieurs substances actives sur un matériau souple ***caractérisé en ce qu'**il* comprend les étapes suivantes□
a) sélectionner un polymère réticulé poreux,
b) incorporer une composition lipophile liquide à la température ambiante, comprenant au moins une substance active, dans le polymère par chauffage à une température supérieure de 1 à 5 °C à la température de transition vitreuse du polymère afin d'obtenir des microsphères chargées,
c) disposer les microsphères chargées sur un matériau souple,
d) chauffer le matériau souple comprenant les microsphères chargées à une température supérieure de 1 à 10 °C à la température de fusion du polymère, sans atteindre l'état de fusion du polymère,
e) obtenir le refroidissement du matériau souple imprégné de microsphères chargées.

2. Procédé selon la revendication 1, ***caractérisé en ce que*** le polymère réticulé est choisi parmi des polyamides, des polyéther block amides ou des éthylène vinyle acétate.

3. Procédé selon la revendication 1, ***caractérisé en ce que*** plusieurs microsphères constituées de différents polymères réticulés poreux choisis parmi des polyamides, des polyéthers block amides ou des éthylènes vinyle acétate sont formées et mélangées au cours de l'étape b).

4. Procédé selon la revendication 1, ***caractérisé en ce que*** la substance active est choisie parmi des composés présentant un effet de nature thérapeutique, dermo-cosmétique ou olfactive.

5. Procédé selon la revendication précédente, ***caractérisé en ce que*** la substance active est choisie parmi des molécules à action anti-douleur, anti-inflammatoire, veinotonique, amincissante -lipolytique et/ou drainante-, hydratante, apaisante, anti-âge -antioxydante et/ou restructurante et/ou réparatrice-, éclaircissante, jambes lourdes, anti-stress, désodorisante%dorisante, attractive et/ou répulsive.

6. Procédé selon la revendication 1, ***caractérisé en ce que*** la composition lipophile liquide à la température ambiante est formulée à partir d'huiles essentielles, d'essences naturelles ou de synthèse, de substances liquides ou solubilisées en milieu lipophile, naturelles ou de synthèse.

7. Procédé selon la revendication 1, ***caractérisé en ce que*** l'étape b) est réalisée en un temps inférieur à 5 minutes.

8. Procédé selon la revendication 1, ***caractérisé en ce que**,* au cours de l'étape b), la substance active est incorporée au polymère réticulé poreux dans un rapport pondéral représentant 1 à 40 % en poids de la microsphère chargée résultante.

9. Procédé selon la revendication 1, ***caractérisé en ce que**,* au cours de l'étape b), plusieurs substances actives de nature différente sont incorporées dans un type de microsphère.

10. Procédé selon la revendication 1, ***caractérisé en ce que*** le matériau souple est constitué par un support fibreux ou poreux sur lequel les microsphères sont susceptibles d'être disposées et fixées par thermo-application sans atteindre l'état de fusion des microsphères.

11. Procédé selon la revendication précédente, ***caractérisé en ce que*** le matériau souple est choisi parmi des textiles tissés ou non-tissés, composés de fibres naturelles d'origine animale ou végétale, ou encore des fibres synthétiques.

12. Procédé selon la revendication 1, ***caractérisé en ce que*** l'étape d) est réalisée en un temps compris entre 1 à 10 secondes.

13. Procédé selon la revendication 1, ***caractérisé en ce que*** l'étape d) comprend plusieurs étapes de chauffage ajustées selon une température supérieure de 1 à 10 °C à la température de fusion du polymère réticulé poreux considéré, en commençant par les températures de fusion les plus hautes pour finir avec les températures de fusion les plus faibles, sans pour autant jamais obtenir la fusion des microsphères.

14. Procédé selon la revendication 1, ***caractérisé en ce* qu'**une force de pression est exercée conjointement au chauffage au cours de l'étape d).

15. Matériau souple enduit de microsphères polymériques chargées en une ou plusieurs substances actives obtenu selon l'une des revendications 1 à 13, ***caractérisé en ce qu'***il se présente sous forme de vêtement, sous-vêtement ou accessoire textile ou poreux pouvant être porté par un utilisateur.

16. Utilisation d'un matériau souple selon la revendication précédente pour la diffusion contrôlée de substances actives en contact direct ou non avec la peau d'un utilisateur.

## Claims

1. Method for coating polymer microspheres laden with one or more active substances onto a flexible material without melting, ***characterised in that*** it comprises the following steps:
a) selecting a porous crosslinked polymer,
b) incorporating a liquid lipophilic composition at room temperature containing at least one active substance into the polymer by heating at a temperature higher by 1 to 5°C than the glass transition temperature of the polymer in order to obtain laden microspheres,
c) placing the laden microspheres on a flexible material,
d) heating the flexible material with the laden microspheres at a temperature higher by 1 to 10°C than the polymer melting temperature, without reaching the molten state of the polymer,
e) cooling the flexible material impregnated with the laden microspheres.

2. Method according to claim 1, ***characterised in that*** the crosslinked polymer is chosen from polyamides, polyether block amides or ethylene vinyl acetates.

3. Method according to claim 1, ***characterised in that*** a plurality of microspheres composed of different porous crosslinked polymers selected from polyamides, polyether block amides or ethylene vinyl acetates are formed and mixed during step b).

4. Method according to claim 1, ***characterised in that*** the active substance is selected from compounds having an effect of a therapeutic, dermatological, cosmetic or olfactory nature.

5. Method according to the preceding claim, ***characterised in that*** the active substance is selected from molecules with analgesic, anti-inflammatory, veinotonic, slimming-lipolytic and/or draining, moisturising, soothing, anti-ageing-antioxidant and/or restructuring and/or repairing, clarifying, heavy legs, anti-stress, deodorising/odorising, attractant and/or repellent action.

6. Method according to claim 1, ***characterised in that*** the lipophilic composition that is liquid at room temperature is formulated from essential oils, natural or synthetic essences, natural or synthetic substances in liquid form or dissolved in a lipophilic medium.

7. Method according to claim 1, ***characterised in that*** step b) is performed in a time of less than 5 minutes.

8. Method according to claim 1, ***characterised in that**,* at step b), the active substance is incorporated into the porous cross-linked polymer in a weight ratio representing 1 to 40% by weight of the resulting laden microsphere.

9. Method according to claim 1, ***characterised in that**,* at step b), several active substances of different nature are incorporated into one type of microsphere.

10. Method according to claim 1, ***characterised in that*** the flexible material comprises a fibrous or porous substrate on which the microspheres are capable of being disposed and fixed by thermal application without the microspheres reaching the melted state.

11. Method according to the preceding claim, ***characterised in that*** the flexible material is selected from woven and non-woven textiles composed of natural fibres of animal or vegetable origin, or synthetic fibres.

12. Method according to claim 1, ***characterised in that*** step d) is performed in a time of between 1 and 10 seconds.

13. Method according to claim 1, ***characterised in that*** step d) includes several heating steps adjusted according to a temperature higher by 1 to 10°C than the melting temperature of the porous cross-linked polymer in question, starting with the highest melting temperatures and ending with the lowest melting temperatures, without in so doing reaching the melted state of the microspheres.

14. Method according to claim 1, ***characterised in that*** a pressure force is exerted in conjunction with the heating during step d).

15. Flexible material coated with polymer microspheres laden with one or more active substances obtained according to any one of claims 1-13, ***characterised in that*** it is presented in the form of garments, under-garments or textile or porous accessories that can be worn by a user.

16. Use of a flexible material according to the preceding claim for the controlled diffusion of active substances in direct contact or otherwise with the skin of a user.

## Patentansprüche

1. Verfahren zum schmelzfreien Auftragen von mit einem oder mehreren Wirkstoffen beladenen polymeren Mikrokügelchen auf ein nachgiebiges Material, **dadurch gekennzeichnet, dass** es folgende Schritte aufweist:
a) Auswählen eines vernetzten, porösen Polymers,
b) Beimengen einer lipophilen, flüssigen Zusammensetzung, die zumindest einen Wirkstoff enthält, zu dem Polymer bei Raumtemperatur durch Erhitzen auf eine Temperatur, die um 1 bis 5 °C höher als die Glasübergangstemperatur des Polymers ist, um beladene Mikrokügelchen zu erhalten,
c) Anordnen der beladenen Mikrokügelchen auf ein nachgiebiges Material,
d) Erhitzen des die beladenen Mikrokügelchen enthaltenden nachgiebigen Materials auf eine Temperatur, die um 1 bis 10 °C höher als die Schmelztemperatur des Polymers ist, ohne dabei den Schmelzzustand des Polymers zu erreichen,
e) Bewirken der Abkühlung des mit beladenen Mikrokügelchen durchzogenen, nachgiebigen Materials.

2. Verfahren nach Anspruch 1, **dadurch gekennzeichnet, dass** das vernetzte Polymer ausgewählt ist aus Polyamiden, Polyether-Block-Amiden oder Ethylenvinylacetaten.

3. Verfahren nach Anspruch 1, **dadurch gekennzeichnet, dass** mehrere Mikrokügelchen, die aus verschiedenen vernetzten, porösen Polymeren, ausgewählt aus Polyamiden, Polyether-Block-Amiden oder Ethylenvinylacetaten, bestehen, gebildet und im Laufe von Schritt b) vermischt werden.

4. Verfahren nach Anspruch 1, **dadurch gekennzeichnet, dass** der Wirkstoff aus Verbindungen ausgewählt wird, die eine therapeutische Wirkung, eine dermo-kosmetische Wirkung oder eine Geruchswirkung haben.

5. Verfahren nach dem vorangehenden Anspruch, **dadurch gekennzeichnet, dass** der Wirkstoff ausgewählt ist aus Molekülen mit schmerzlindernder Wirkung, entzündungshemmender Wirkung, Wirkung als Venentonikum, als Schlankheitsmittel - fettspaltend und/oder entschlackend - mit feuchtigkeitsspendender Wirkung, beruhigender Wirkung, Anti-Aging-Wirkung - antioxidativ und/oder restrukturierend und/oder reparierend - mit aufhellender Wirkung, mit Wirkung gegen schwere Beine, mit Antistress-Wirkung, mit desodorisierender/odorisierender Wirkung, mit anziehender und/oder abstoßender Wirkung.

6. Verfahren nach Anspruch 1, **dadurch gekennzeichnet, dass** die bei Raumtemperatur flüssige lipophile Zusammensetzung ausgehend von ätherischen Ölen, natürlichen oder synthetischen Essenzen, flüssigen oder in lipophilem Milieu löslich gemachten, natürlichen oder synthetischen Substanzen gebildet wird.

7. Verfahren nach Anspruch 1, **dadurch gekennzeichnet, dass** der Schritt b) in einer Zeit von unter 5 Minuten durchgeführt wird.

8. Verfahren nach Anspruch 1, **dadurch gekennzeichnet, dass** im Laufe von Schritt b) der Wirkstoff dem vernetzten, porösen Polymer beigemengt wird, und zwar in einem Gewichtsverhältnis von 1 bis 40 Gew.-% des resultierenden beladenen Mikrokügelchens.

9. Verfahren nach Anspruch 1, **dadurch gekennzeichnet, dass** im Laufe von Schritt b) mehrere Wirkstoffe unterschiedlicher Art einer Art von Mikrokügelchen beigemengt werden.

10. Verfahren nach Anspruch 1, **dadurch gekennzeichnet, dass** das nachgiebige Material aus einem faserigen oder porösen Träger besteht, auf dem die Mikrokügelchen angeordnet und durch Thermoanwendung fixiert werden können, ohne dabei den Schmelzzustand der Mikrokügelchen zu erreichen.

11. Verfahren nach dem vorangehenden Anspruch, **dadurch gekennzeichnet, dass** das nachgiebige Material ausgewählt ist aus gewebten oder nichtgewebten Textilerzeugnissen, die aus natürlichen Fasern tierischer oder pflanzlicher Herkunft oder auch aus Synthetikfasern bestehen

12. Verfahren nach Anspruch 1, **dadurch gekennzeichnet, dass** der Schritt d) in einer Zeit von zwischen 1 bis 10 Sekunden durchgeführt wird.

13. Verfahren nach Anspruch 1, **dadurch gekennzeichnet, dass** der Schritt d) mehrere Heizschritte aufweist, die gemäß einer Temperatur um 1 bis 10 °C höher als die Schmelztemperatur des betrachteten vernetzten, porösen Polymers, ausgehend von den höchsten Schmelztemperaturen bis hin zu den niedrigsten Schmelztemperaturen, angeglichen werden, jedoch ohne dabei jemals das Schmelzen der Mikrokügelchen zu erreichen.

14. Verfahren nach Anspruch 1, **dadurch gekennzeichnet, dass** zusammen mit dem Erhitzen im Laufe von Schritt d) eine Druckkraft ausgeübt wird.

15. Nachgiebiges Material, das mit polymeren Mikrokügelchen, die mit einem oder mehreren Wirkstoffen beladen sind, beschichtet ist und nach einem der Ansprüche 1 bis 13 erhalten wird, **dadurch gekennzeichnet, dass** es in Form von Kleidung, Unterkleidung oder textilem oder porösem Textilzubehörteil vorliegt, die/das von einem Benutzer getragen werden kann.

16. Verwendung eines nachgiebigen Materials nach dem vorangehenden Anspruch für die kontrollierte Diffusion von Wirkstoffen in mittelbarem oder unmittelbarem Kontakt mit der Haut eines Benutzers.
